# EUROPÄISCHE PATENTANMELDUNG

(11) **EP 3 381 365 A1**
(43) Veröffentlichungstag der Anmeldung: **03.10.2018**
(21) Anmeldenummer: 18163515.2
(22) Anmeldetag: 23.03.2018
(51) Int. Cl.: A61B 5/08, G01N 33/497

(54) **EINRICHTUNG ZUR AUFNAHME UND SPÜLUNG EINER VORRICHTUNG ZUR ATEMGASANALYSE**

(30) Priorität: 29.03.2017 DE 102017205292
(71) Anmelder: Robert Bosch GmbH, 70442 Stuttgart (DE)
(72) Erfinder: Erckert, Beatrix, 70378 Stuttgart (DE); Thuersam, Markus, 71263 Weil Der Stadt (DE)

(57) **Zusammenfassung**

Die Erfindung betrifft eine Einrichtung (100) und ein Verfahren (500) zur Aufnahme und Spülung einer Vorrichtung (10) zur Messung von Analyten in Ausatemluft, wobei die Einrichtung (100) ein Anschlusselement (110) zum Verbinden der Einrichtung (100) mit einem Messpfad der Vorrichtung (10) und eine Spüleinheit (140) zum Einleiten von Spülfluid über das Anschlusselement (110) in den Messpfad (11) aufweist.

## Beschreibung

### Stand der Technik

Über quantitative Messungen von Analyten in Ausatemluft können bestimmte Atemwegserkrankungen erkannt und überwacht werden. Beispielsweise kann über die Bestimmung der Stickstoffmonoxidkonzentration in ausgeatmeter Luft ein Maß für die Entzündung der Lunge bei Asthma abgeschätzt werden.

Die Bestimmung der Stickstoffmonoxidkonzentration kann dabei mit einer in EP 1384069 B1 offenbarten Vorrichtung über die Konversion von Stickstoffmonoxid zu Stickstoffdioxid mit nachfolgender Messung der Stickstoffdioxidkonzentration mithilfe eines feldeffektransistorbasierten Gassensors in der Vorrichtung erfolgen. Für die Regeneration des Gassensors nach einer Messung ist eine Spülung mit einem Stickstoffdioxid-freien Gasgemisch erforderlich. In EP 1384069 B1 wird vorgeschlagen, Umgebungsluft über einen Stickoxid-Filter von Stickstoffmonoxid und -dioxid zu reinigen und dem Gassensor zuzuführen. Der Stickoxid-Filter, auch Scrubber genannt, befindet sich dabei in der Vorrichtung, beispielsweise in einem austauschbaren Mundstück der Vorrichtung. Dazu ist neben einem Messpfad ohne Scrubber ein separater Spülpfad mit Scrubber in der Vorrichtung bzw. in dem Mundstück der Vorrichtung erforderlich.

### Offenbarung der Erfindung

### Vorteile der Erfindung

Vor diesem Hintergrund betrifft die Erfindung eine Einrichtung zur Aufnahme und Spülung einer Vorrichtung zur Messung von Analyten in Ausatemluft. Die Einrichtung weist ein Anschlusselement zum Verbinden der Einrichtung mit einem Messpfad der Vorrichtung auf. Ferner umfasst die Einrichtung eine Spüleinheit zum Einleiten von Spülfluid über das Anschlusselement in den Messpfad der Vorrichtung.

Unter Ausatemluft kann insbesondere von einem Benutzer der Vorrichtung ausgeatmete Luft, auch Ausatemgas oder Atemgas genannt, verstanden werden. Bei der Vorrichtung zur Messung von Analyten in Ausatemluft kann es sich somit um ein Gerät zur Atemgasanalyse handeln, beispielsweise um eine Vorrichtung zur Messung von Stickstoffmonoxid in Ausatemluft basierend auf einer Konversion von Stickstoffmonoxid zu Stickstoffdioxid und einer Messung der Stickstoffdioxidkonzentration. Unter einem Analyten kann insbesondere eine chemische Spezies wie beispielweise eine in der Ausatemluft vorkommende Spezies wie Stickstoffmonoxid oder Stickstoffdioxid verstanden werden. Unter den Begriff Analyt können auch Partikel oder biologische Entitäten wie beispielsweise Proteine fallen. Mit einer Aufnahme der Vorrichtung in der Einrichtung kann insbesondere eine Verbindung der Einrichtung mit der Vorrichtung verstanden werden, insbesondere über das Anschlusselement der Einrichtung. Beispielsweise kann die Einrichtung eine Ausnehmung aufweisen, in welche zumindest ein Teil der Vorrichtung aufgenommen werden kann. Bei dem Anschlusselement kann es sich beispielsweise um einen Teil der Einrichtung handeln, der mit einem der Teil der Vorrichtung formschlüssig und vorzugsweise luftdicht verbunden werden kann. Unter Messpfad kann insbesondere eine Leitung oder ein Kanal für eine Fluid, insbesondere Ausatemluft, zu einem Sensor, insbesondere einem Gassensor, der Vorrichtung verstanden werden. Bei dem Spülfluid kann es sich insbesondere um ein Gas oder Gasgemisch wie Luft oder um eine Flüssigkeit handeln.

Die Einrichtung hat den Vorteil, dass die Spülfunktionalität aus der Vorrichtung in die Einrichtung verlagert wird und dadurch die Vorrichtung kompakter und weniger aufwendig ausgeführt werden kann. Ferner ist von Vorteil, dass eine Spülung der Vorrichtung nicht im Zuge des Betriebs der Vorrichtung erfolgen muss und somit Zeit und Ressourcen, insbesondere in der Vorrichtung bereitgestellte Energie, eingespart werden können. Darüber hinaus ist von Vorteil, dass eine Spülung der Vorrichtung während einer Lagerung der Vorrichtung gemeinsam mit der Einrichtung erfolgen kann. Eine Zeitdauer der Nichtbenutzung der Vorrichtung für eine Messung kann somit vorteilhafterweise zur Vorbereitung einer nächsten Messung genutzt werden.

Wie oben ausgeführt kann die Einrichtung eine Ausnehmung für eine vorzugsweise formschlüssige Aufnahme der Vorrichtung, insbesondere für eine formschlüssige Aufnahme eines mit dem Anschlusselement verbindbaren Teils der Vorrichtung, aufweisen. Dies ermöglicht eine besonders einfache und fehlersichere Aufnahme der Vorrichtung, da ein Benutzer aufgrund des Formschlusses zu einer korrekten Verbindung der Einrichtung mit der Vorrichtung angeleitet und geführt wird.

Bevorzugt ist das Anschlusselement in Form einer oder als Teil einer die Ausnehmung begrenzenden Erhebung ausgebildet. So kann die Vorrichtung besonders einfach und sicher in der Ausnehmung über eine Verbindung mit dem Anschlusselement verankert werden.

Beispielsweise ist das Anschlusselement in Form einer Clipverbindung ausgeführt, so dass der mit dem Anschlusselement zu verbindende Teil der Vorrichtung auf einfache Weise in das Anschlusselement eingeführt werden kann.

Das Anschlusselement kann ferner ein Ventil aufweisen, insbesondere ein Rückschlagventil. Dies stellt vorteilhafterweise sicher, dass nur bei einer Verbindung der Vorrichtung mit dem Anschlusselement Fluid aus dem Anschlusselement austreten kann.

Die Einrichtung zur Aufnahme und Spülung der Vorrichtung kann beispielsweise die Form eines Etuis zur Aufbewahrung der Vorrichtung aufweisen, beispielsweise in Form einer Schale. Dabei kann es sich um ein Etui handeln, das eine vollständige Aufnahme und Umschließung der Vorrichtung und somit eine vor äußeren Einwirkungen geschützte Verwahrung der Vorrichtung ermöglicht.

Ferner kann die Einrichtung als Ladeeinrichtung oder Ladestation für die Vorrichtung ausgebildet sind, insbesondere zur elektrischen Aufladung eines Energiespeichers der Vorrichtung. Dies hat den Vorteil, dass die beiden Funktionen des Aufladens und Spülens in einer gemeinsamen Einrichtung kompakt untergebracht werden können. Vorzugsweise umfasst die Einrichtung dabei eine Ladeeinheit zur induktiven elektrischen Energieübertragung an die Vorrichtung. Somit wird vorteilhafterweise kein separater elektrischer Anschluss an die Vorrichtung benötigt.

Vorzugsweise weist die Spüleinheit eine Pumpe auf, wobei die Pumpe ausgebildet ist, das Spülfluid über das Anschlusselement in den Messpfad der Vorrichtung zu pumpen. Dies hat den Vorteil, dass auf ein Ansaugen des Spülfluids von Seiten der Vorrichtung verzichtet werden kann. Dies erlaubt es, die Pumpleistung einer Pumpe in der Vorrichtung nicht übermäßig zu beanspruchen oder gar vollständig auf eine solche Pumpe in der Vorrichtung zu verzichten.

In einer vorteilhaften Weiterbildung der Erfindung umfasst die Spüleinheit einen Filter zur Filterung von Partikeln oder chemischer Spezies, insbesondere in der Ausatemluft vorhandener Analyten wie Stickoxide, beispielsweise Stickstoffdioxid, und/oder Partikel aus dem Spülfluid. Der Filter kann dafür Aktivkohle, Aluminiumoxid, Kaliumpermanganat, Zeolith und/oder Kieselgel umfassen. Dies ist besonders vorteilhaft, da durch den Filter gewährleistet wird, dass das Spülfluid vor dem Einleiten in den Messpfad der Vorrichtung von für eine Messung beeinträchtigende Partikel oder Spezies gereinigt wird. Insbesondere wird durch eine Filterung von in der Ausatemluft enthaltenen Analyten aus dem Spülfluid verhindert, dass solche Analyten vor einer Messung in die Vorrichtung gelangen und eine spätere Messung verfälschen.

Gemäß einer vorteilhaften Ausgestaltung der Erfindung weist die Einrichtung ein Ventil zur Aufnahme von Luft aus einer Umgebung der Einrichtung auf. Das Ventil ist dabei mit der Spüleinheit der Vorrichtung verbunden, so dass eine über das Ventil aufgenommene Luft als Spülfluid verwendet werden kann, vorzugsweise nach einer Filterung wie oben beschrieben. Bevorzugt weist die Einrichtung, insbesondere die Spüleinheit, eine Pumpe auf, welche eingerichtet ist, Luft über das Ventil in die Einrichtung einzusaugen. Vorzugsweise kann es sich dabei um dieselbe oben beschriebene Pumpe handeln, welche für die Leitung des Fluids in den Messpfad der Vorrichtung eingerichtet ist.

In einer weiteren vorteilhaften Ausgestaltung umfasst die Einrichtung einen Vorratsbehälter für Spülfluid, beispielsweise in Form einer Gaspatrone, wobei der Vorratsbehälter mit der Spüleinheit fluidisch verbunden ist. Dies ermöglicht es, bereits geeignetes Spülfluid in der Einrichtung selbst vorzulagern.

In einer vorteilhaften Weiterbildung der Erfindung umfasst die Einrichtung eine, insbesondere drahtlose, Kommunikationsschnittstelle zur Vorrichtung, insbesondere für eine Steuerung eines Regenerationsverfahrens für einen Sensor in der Vorrichtung. Dadurch kann vorteilhafterweise im Zuge einer Spülung des Messpfades der Vorrichtung auch der Sensor, insbesondere der Gassensor, regeneriert werden. Beispielweise kann das Regenerationsverfahren eine Erhitzung des Gassensor für eine Desorption unerwünschter Spezies oder Analyten aus einer sensitiven Schicht des Gassensors umfassen, wobei durch eine gleichzeitige Spülung die Desorption und Wegbeförderung der adsorbierten Spezies oder Analyten verbessert wird.

Gegenstand der Erfindung ist auch ein Verfahren zur Spülung einer Vorrichtung zur Messung von Analyten in Ausatemluft. In einem ersten Schritt wird die Vorrichtung mit einer Einrichtung zur Spülung der Vorrichtung verbunden, wobei die Vorrichtung über ein Anschlusselement der Einrichtung mit der Vorrichtung verbunden wird. In einem zweiten Schritt erfolgt ein Einleiten von Spülfluid in einen Messpfad der Vorrichtung über eine Spüleinheit der Einrichtung, insbesondere zur Spülung eines Sensors der Vorrichtung.

In einer vorteilhaften Ausgestaltung des Verfahrens wird das Spülfluid vor dem Einleiten in die Vorrichtung durch einen Filter zur Filterung von Partikeln oder Spezies, insbesondere Stickstoffdioxid, geleitet. Dabei kann es sich um einen oben beschriebenen Filter handeln.

In einer vorteilhaften Weiterbildung des Verfahrens wird zumindest ein Teil des Sensors der Vorrichtung auf eine Regenerationstemperatur aufgeheizt. Wie oben beschrieben, unterstützt dies eine Entfernung unerwünschter Partikel, Spezies oder Analyten aus dem Messpfad und von einem im Messpfad angeordneten Sensor.

### Kurze Beschreibung der Zeichnungen

Ausführungsbeispiele der Erfindung sind in den Zeichnungen schematisch dargestellt und in der nachfolgenden Beschreibung näher erläutert. Für die in den verschiedenen Figuren dargestellten und ähnlich wirkenden Elemente werden gleiche Bezugszeichen verwendet, wobei auf eine wiederholte Beschreibung der Elemente verzichtet wird.

Es zeigen
- Figur 1: ein erstes Ausführungsbeispiel der erfindungsgemäßen Einrichtung,
- Figur 2: ein zweites Ausführungsbeispiel der erfindungsgemäßen Einrichtung und
- Figur 3: ein Ablaufdiagramm eines Ausführungsbeispiels des erfindungsgemäßen Verfahrens,

### Ausführungsformen der Erfindung

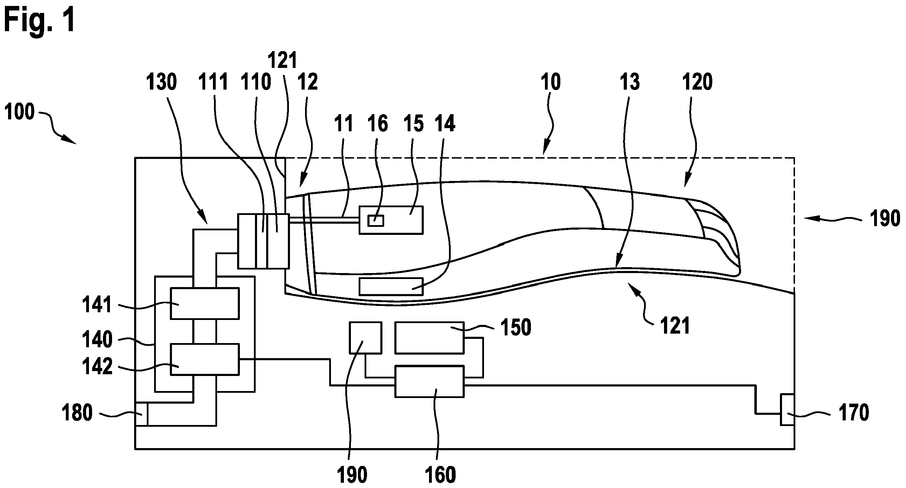

Figur 1 zeigt ein Ausführungsbeispiel der erfindungsgemäßen Einrichtung 100 zur Aufnahme und Spülung einer Vorrichtung 10 zur Messung von Analyten in Ausatemluft. Die Einrichtung 100 weist ein Anschlusselement 110 zum Verbinden der Einrichtung 100 mit einem Messpfad 11 der Vorrichtung 10 und eine Spüleinheit 140 zum Einleiten von Spülfluid über das Anschlusselement 110 in den Messpfad 11 auf. Bei der Vorrichtung 10 zur Messung von Analyten in Ausatemluft kann es beispielsweise um eine Vorrichtung zur Messung von Stickstoffmonoxid in Ausatemluft basierend auf einer Konversion von Stickstoffmonoxid zu Stickstoffdioxid und einer Messung der Stickstoffdioxidkonzentration handeln. Dazu kann die Vorrichtung 10 eine mit einem Messpfad 11 verbundene Messkammer 15 umfassen, in welcher sich ein Sensor 16 zur Messung der Stickstoffdioxidkonzentration befindet. Bei dem Sensor 16 kann sich insbesondere um einen feldeffektransistorbasierten Gassensor handeln.

Wie aus Figur 1 ersichtlich weist die Einrichtung 100 eine Ausnehmung 120 für eine formschlüssige Aufnahme der Vorrichtung 10 auf, in dem eine Oberfläche der Einrichtung 121 derart gebogen ist, dass sie sich bei einer Verbindung des Anschlusselements 110 mit einem mit dem Anschlusselement 110 verbindbaren Teil 12 der Vorrichtung 10 an eine Außenfläche 13 der Vorrichtung formschlüssig anschmiegt. Insbesondere kann dabei der mit dem mit dem Anschlusselement 110 verbindbare Teil 12 der Vorrichtung 10 formschlüssig aufgenommen werden. Die Verbindung des Teils 12 mit dem Anschlusselement 110 für eine Verbindung des Anschlusselements 110 mit dem Messpfad 11 der Vorrichtung 10 erfolgt dabei beispielsweise über eine Clipverbindung, beispielsweise indem der Teil 12 einen Abschnitt des Anschlusselements 110 aufnimmt, wie in Figur 1 gezeigt. Wie ebenfalls in Figur ersichtlich, ist das Anschlusselement 110 als Teil einer die Ausnehmung 120 begrenzenden Erhebung 121 ausgebildet. Diese Begrenzung 121 unterstützt die sichere Aufnahme der Vorrichtung 10 in die Ausnehmung 120.

In einer bevorzugten Ausgestaltung umfasst die Einrichtung 100 eine entfernbare Abdeckung 190 der Ausnehmung 120, welche in Figur 1 durch eine gebrochene Linie 190 dargestellt wird. Dadurch wird eine in der Ausnehmung 120 aufgenommene Vorrichtung 10 vor äußeren Einwirkungen geschützt. Die Abdeckung 190 kann beispielsweise in Form eines Klappverschlusses ausgeführt sein, so dass die Einrichtung 100 in Form eines Etuis zur Aufbewahrung der Vorrichtung 10 ausgebildet ist.

Im Ausführungsbeispiel gemäß Figur 1 ist die Einrichtung 100 als Ladeeinrichtung, auch Ladestation oder Ladeschale genannt, für die Vorrichtung 10 ausgebildet. Die Einrichtung 100 weist eine Ladeeinheit 150 zur Aufladung eines Energiespeichers 14, beispielsweise eines Akkumulators, der Vorrichtung 10 auf. Die Ladeeinheit 150 kann dabei, wie in Figur 1 angedeutet, zur induktiven Aufladung des Energiespeichers 14 ausgebildet sein. Alternativ können sowohl die Einrichtung 100 als auch die Vorrichtung 10 elektrische Kontakte zur Aufladung des Energiespeichers 14 umfassen. Die Ladeeinheit 150 kann über eine Steuereinheit oder Steuergerät 160 der Einrichtung angesteuert werden. Das Steuergerät 160 kann eingerichtet sein, bei einer über einen Sensor 16, insbesondere einem Annährungssensor 16, der Einrichtung festgestellten Aufnahme der Vorrichtung 10 die Aufladung des Energiespeichers 14 über die Ladeeinheit 150 anzusteuern. Alternativ kann das Steuergerät 160 eingerichtet sein, über eine manuelle Bedienung eines Benutzers eine Ansteuerung der Ladeeinheit 150 zu bewirken. Das Steuergerät 160 kann eine Verbindung 170 für eine externe Bestromung und/oder für eine Datenübertragung aufweisen, beispielsweise in Form eines USB-Anschlusses 170.

Wie oben beschrieben, ist die Spüleinheit 140 der Einrichtung 100 mit dem Anschlusselement 110 über einen Fluidkanal 130 verbunden. Die Spüleinheit 140 weist einen Filter 141 zur Filterung von Partikeln und/oder Spezies, insbesondere Stickstoffdioxid auf. Beispielsweise kann der Filter 141 Aktivkohle, Aluminiumoxid, Kaliumpermanganat, Zeolith und/oder Kieselgel umfassen, insbesondere zur Filterung von Stickoxiden, beispielsweise Stickstoffdioxid. Ferner weist die Spüleinheit 140 eine Pumpe 142 auf, wobei die Pumpe 142 ausgebildet ist, Spülfluid über das Anschlusselement 110 in den Messpfad 11 der Vorrichtung 10 zu leiten. Beispielsweise handelt es sich bei der Pumpe 142 um eine Mikropumpe. In diesem Ausführungsbeispiel ist die Spüleinheit 140 mit einem Ventil 180 zur Aufnahme von Luft aus einer Umgebung der Einrichtung 100 verbunden, so dass Luft als Grundlage für das Spülfluid verwendet werden kann. Die Luft kann somit über die Pumpe 142 angesaugt und über den Filter 142 zum Anschlusselement 110 für eine Weiterleitung in den Messpfad 11 der Vorrichtung 10 gepumpt werden. Vorzugsweise umfasst das Anschlusselement 110 ein Ventil 111, welches nur bei einer Verbindung des Anschlusselements 110 mit dem Messpfad 11 der Vorrichtung 10 geöffnet wird, beispielsweise über eine Ansteuerung durch das dazu eingerichtete Steuergerät 160. Ferner kann das Steuergerät 160 eingerichtet sein, eine Spülung der Vorrichtung 10 zu veranlassen, wenn eine Verbindung der Vorrichtung 10 mit dem Anschlusselement 110 festgestellt wird, beispielsweise über den oben genannten Sensor 16. Somit kann vorteilhafterweise eine Spülung des Sensors 16 in der Vorrichtung 10 während einer Aufbewahrung der Vorrichtung 10 in der Ausnehmung 120 der Einrichtung 100 erfolgen.

In einer besonderes vorteilhaften Ausgestaltung der Einrichtung 100 umfasst die Einrichtung 100 eine, insbesondere drahtlose, Kommunikationsschnittstelle 190 für eine Kommunikation mit der Vorrichtung 10, beispielsweise eine Bluetooth®-, NFC- und/oder WLAN-Schnittstelle. Das Steuergerät 160 kann dabei gemeinsam mit der Kommunikationsschnittstelle 190 eingerichtet sein, um mit einer entsprechenden Schnittstelle in der Vorrichtung 10 zu kommunizieren. Beispielsweise kann dadurch eine Verbindung der Vorrichtung 10 mit dem Anschlusselement 110 erkannt werden. Vorzugsweise kann die Kommunikationsschnittstelle 190, beispielsweise gemeinsam mit dem Steuergerät 160, für eine Steuerung eines Regenerationsverfahrens für den Sensor 16 in der Vorrichtung 10 eingerichtet sein. Das Regenerationsverfahren kann dabei beispielsweise eine Aufheizung zumindest eines Teils des Sensors 16 für eine Desorption von an einer Oberfläche oder an einer sensitiven Schicht des Sensors 16 gebundenen unerwünschten Spezies, Partikel oder Analyten umfassen. Insbesondere kann das Steuergerät 160 eingerichtet sein, über die Kommunikationsschnittstelle 190 eine Aufheizung des Sensors 16 im Zusammenhang mit einer Ansteuerung der Spüleinheit 140 zu bewirken. Beispielsweise kann bei Verwendung eines in EP1384069B1 beschriebenen Gassensors die sensitive Schicht auf 95 °C aufgeheizt werden.

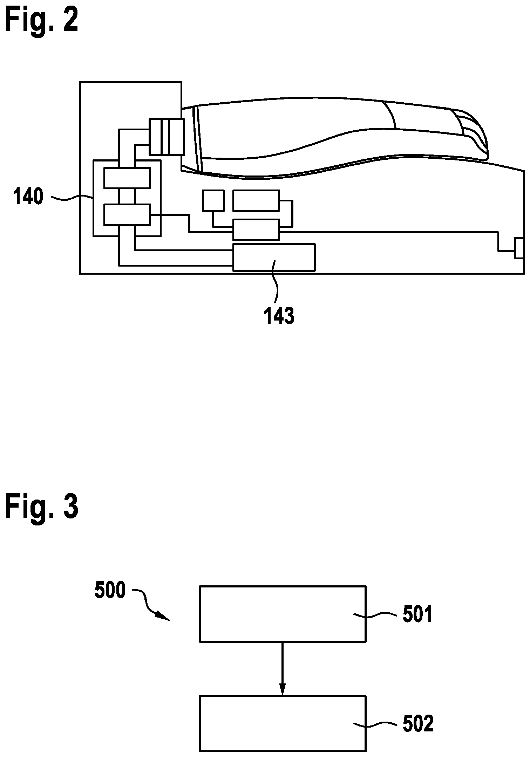

Figur 2 zeigt ein zweites Ausführungsbeispiel der erfindungsgemäßen Einrichtung 100, welches sich von dem ersten Ausführungsbeispiel nur durch eine alternative Quelle für das Spülfluid unterscheidet. In diesem Beispiel ist die Spüleinheit 140 mit einem Vorratsbehälter 143 für Spülfluid fluidisch verbunden. Der Vorratsbehälter 143 kann beispielsweise eine Gaspatrone umfassen, in welcher Spülfluid in Form eines Gasgemischs, beispielsweise eine Mischung aus Sauerstoff und Stickstoff, vorgelagert ist. Somit kann in dieser Variante auf Umgebungsluft zur Spülung des Sensors 16 der Vorrichtung 10 verzichtet werden.

Figur 3 zeigt ein Ablaufdiagramm eines Ausführungsbeispiels des erfindungsgemäßen Verfahrens 500 zur Spülung einer Vorrichtung zur Messung von Analyten in Ausatemluft, beispielsweise unter Verwendung der Einrichtung 100 und der Vorrichtung 10 aus den oben beschriebenen Ausführungsbeispielen.

In einem ersten Schritt 501 des Verfahrens wird die Vorrichtung 10 mit einer Einrichtung 100 zur Spülung der Vorrichtung 10 verbunden, wobei die Verbindung der Vorrichtung 10 über ein Anschlusselement 110 der Einrichtung 100 mit der Vorrichtung 10 erfolgt. In einem zweiten Schritt 503 wird Spülfluid in einen Messpfad 11 der Vorrichtung 10 über eine Spüleinheit 140 der Einrichtung 100 eingeleitet, insbesondere zur Spülung eines Sensors 16 der Vorrichtung 10. Vorzugsweise wird das Spülfluid vor dem Einleiten in die Vorrichtung 10 durch einen Filter 141 zur Filterung von Partikeln und/oder Spezies, insbesondere Stickstoffdioxid, aus dem Spülfluid geleitet. Ferner wird, wie oben beschrieben, vorzugsweise zumindest ein Teil des Sensors 16 der Vorrichtung 10 auf eine Regenerationstemperatur aufgeheizt.

## Patentansprüche

1. Einrichtung (100) zur Aufnahme und Spülung einer Vorrichtung (10) zur Messung von Analyten in Ausatemluft, wobei die Einrichtung (100) ein Anschlusselement (110) zum Verbinden der Einrichtung (100) mit einem Messpfad der Vorrichtung (10) und eine Spüleinheit (140) zum Einleiten von Spülfluid über das Anschlusselement (110) in den Messpfad (11) aufweist.

2. Einrichtung (100) nach einem der vorhergehenden Ansprüche, wobei die Spüleinheit (140) einen Filter (141) zur Filterung von Partikeln und/oder Spezies, insbesondere Stickstoffdioxid, aus dem Spülfluid umfasst.

3. Einrichtung (100) nach einem der vorhergehenden Ansprüche, wobei die Spüleinheit (140) eine Pumpe (142) aufweist, wobei die Pumpe (142) ausgebildet ist, das Spülfluid über das Anschlusselement (110) in den Messpfad (11) der Vorrichtung (10) zu pumpen.

4. Einrichtung (100) nach einem der vorhergehenden Ansprüche, wobei die Einrichtung (100) eine Ausnehmung (120) für eine Aufnahme der Vorrichtung (10), insbesondere für eine formschlüssige Aufnahme eines mit dem Anschlusselement (110) verbindbaren Teils (12) der Vorrichtung (10), aufweist.

5. Einrichtung (100) nach Anspruch 4, wobei das Anschlusselement (110) in Form einer oder als Teil einer die Ausnehmung (120) begrenzenden Erhebung (121) ausgebildet ist.

6. Einrichtung (100) nach einem der vorhergehenden Ansprüche, wobei die Einrichtung (100) ein Ventil (180) zur Aufnahme von Luft aus einer Umgebung der Einrichtung (100) als Grundlage für das Spülfluid aufweist.

7. Einrichtung (100) nach einem der vorhergehenden Ansprüche, wobei die Einrichtung (100) einen Vorratsbehälter (143) für Spülfluid aufweist.

8. Einrichtung (100) nach einem der vorhergehenden Ansprüche, wobei die Einrichtung (100) die Form eines Etuis zur Aufbewahrung der Vorrichtung (10) aufweist.

9. Einrichtung (100) nach einem der vorhergehenden Ansprüche, wobei die Einrichtung (100) als Ladeeinrichtung für die Vorrichtung (10) ausgebildet ist und eine Ladeeinheit (150) zur vorzugsweise induktiven elektrischen Energieübertragung an die Vorrichtung (10) aufweist.

10. Einrichtung (100) nach einem der vorhergehenden Ansprüche, wobei die Einrichtung (100) eine, insbesondere drahtlose, Kommunikationsschnittstelle (190) zur Vorrichtung (10) aufweist, insbesondere für eine Steuerung eines Regenerationsverfahrens für einen Sensor (16) in der Vorrichtung (10).

11. Verfahren (500) zur Spülung einer Vorrichtung (10) zur Messung von Analyten in Ausatemluft, umfassend die Schritte:
• Verbinden (501) der Vorrichtung (10) mit einer Einrichtung (100) zur Spülung der Vorrichtung (10), wobei die Vorrichtung (10) über ein Anschlusselement (110) der Einrichtung (100) mit der Vorrichtung (10) verbunden wird.
• Einleiten (502) von Spülfluid in einen Messpfad (11) der Vorrichtung (10) über eine Spüleinheit (140) der Einrichtung (100), insbesondere zur Spülung eines Sensors (16) der Vorrichtung (10).

12. Verfahren (500) nach Anspruch 11, wobei das Spülfluid vor dem Einleiten in die Vorrichtung (10) durch einen Filter (141) zur Filterung von Partikeln und/oder Spezies, insbesondere Stickstoffdioxid, aus dem Spülfluid geleitet wird.

13. Verfahren (500) nach Anspruch 11 oder 12, wobei zumindest ein Teil des Sensors (16) der Vorrichtung (10) auf eine Regenerationstemperatur aufgeheizt wird.
